# FASCICULE DE BREVET EUROPEEN

(11) **EP 1 800 710 B1**
(45) Date de publication et mention de la délivrance du brevet: **20.01.2010**
(21) Numéro de dépôt: 06292006.1
(22) Date de dépôt: 21.12.2006
(51) Int. Cl.: A61N 1/368, A61N 1/372

(54) **Dispositif médical implantable actif tel que stimulateur cardiaque, défibrillateur et/ou cardioverteur de type AAI/DDD, coprenant des moyens de réglage automatique de mode après détection automatique de l'implantation**
Aktive implantierbare medizinische Vorrichtung, insbesondere Herzschrittmacher, Defibrillator, und/oder Kardiovertierer des AAI/DDD-Typs, mit Mitteln zur automatischen Moduseinstellung nach automatischer Implantierungsdetektion
Active implantable medical device such as pacemaker, defibrillator and/or cardiovertor of the AAI/DDD type, comprising automatic mode setting means upon automatic implantation detection

(30) Priorité: 21.12.2005 FR 0513022
(43) Date de publication de la demande: 27.06.2007
(73) Titulaire: ELA MEDICAL, 92541 Montrouge (FR)
(72) Inventeur: Amblard, Amel, 92290 Chatenay Malabry (FR)
(74) Mandataire: Dupuis-Latour, Dominique

(56) Documents cités:
- EP-A- 0 488 904
- EP-A- 1 050 320
- EP-A- 1 438 985
- EP-A- 1 550 480
- US-A- 5 476 485
- US-A- 6 154 675

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation, de cardio-version et/ou de défibrillation en cas de trouble du rythme détecté par le dispositif.

Elle concerne plus particulièrement ceux de ces dispositifs qui comprennent des circuits de stimulation et de détection à la fois sur l'oreillette et sur le ventricule et qui peuvent opérer selon deux modes de fonctionnement, DDD ou AAI. Ces dispositifs peuvent être pourvus d'une fonctionnalité dénommée "AAISafeR" assurant une commutation automatique de mode, de DDD à AAI et inversement.

Le mode de fonctionnement de base d'un tel stimulateur "AAISafeR" est un mode AAI, avec une stimulation auriculaire simple chambre et une surveillance (détection) de l'activité ventriculaire. Ce mode est maintenu tant que la conduction auriculo-ventriculaire est normale, c'est-à-dire tant que chaque événement auriculaire (détection auriculaire, correspondant à une activité spontanée, ou stimulation auriculaire) est suivi d'une détection ventriculaire associée.

Dans certaines circonstances peuvent cependant apparaître des blocs auriculo-ventriculaires (BAV) entraînant un trouble temporaire de dépolarisation du ventricule. Dans ce cas, le stimulateur bascule automatiquement en mode DDD automatique, avec des paramètres (délai AV de stimulation et de détection,...) optimisés pour cette situation de BAV temporaire. Après disparition du BAV, et donc rétablissement de la conduction auriculo-ventriculaire, dès lors qu'un certain nombre de conditions sont remplies le stimulateur retourne automatiquement au mode AAI.

Ce principe de fonctionnement autorise le maintien d'une activité spontanée ventriculaire, pour des patients présentant une conduction atrio-ventriculaire normale la majorité du temps.

Cette commutation automatique entre modes DDD et AAI est notamment décrite dans les EP-A-0 488 904, EP-A-1 346 750, EP-A-1 470 836 et EP-A-1 550 480, tous au nom de ELA Medical.

Le point de départ de l'invention réside dans un certain nombre d'observations effectuées lors du suivi clinique de patients, peu après que ces patients aient reçu un dispositif pourvu d'une telle fonctionnalité "AAISafeR" (dispositif DDD/AAI à commutation automatique de mode).

En effet, ces appareils sont paramétrés en usine soit en mode DDD automatique (sans commutation de mode), soit en mode DDD/AAI à commutation automatique de mode (fonctionnalité "AAISafeR" activée), à charge pour le praticien de modifier si nécessaire le paramétrage standard au moment de l'implantation, en fonction de la pathologie particulière du patient.

Cependant, pour des appareils paramétrés en AAISafeR, l'absence de reprogrammation par le praticien peut avoir des conséquences indésirables chez certains patients chez qui elle serait nécessaire.

En effet, avant l'intervention, le dispositif ne comprend que le seul boîtier du générateur, qui n'est pas encore pourvu de sa sonde.

La sonde n'étant pas connectée, le dispositif ne détectera aucune conduction auriculo-ventriculaire (aucun signal ventriculaire n'est recueilli, puisque la sonde n'est pas encore connectée). Si la fonctionnalité AAISafeR est activée, ceci aura pour effet,de provoquer un basculement en mode DDD, d'abord temporaire, puis prolongé avec un test périodique pour autoriser un retour en AAI

Ce test est exécuté après écoulement d'un délai relativement long après le basculement vers le mode DDD, de manière à éviter des alternances trop rapides de commutations AAI→DDD puis DDD→AAI.

Ainsi, chez les patients appareillés de cette manière, le mode AAISafeR permet un retour en AAI si l'appareil détecte une activité ventriculaire soutenue ou après le test périodique qui interviendra régulièrement, typiquement tous les matins.

Or un tel test, propre à la fonctionnalité AAISafeR, n'a de sens que pour des patients présentant une conduction auriculo-ventriculaire permanente ou quasi-permanente.

En revanche, pour des patients présentant un bloc auriculo-ventriculaire permanent avéré, un tel test, qui génère des cycles ventriculaires longs en acceptant l'absence d'activité ventriculaire pendant une durée de temps limitée, n'est pas approprié. Il peut même s'avérer délétère, en créant chez certains patients vulnérables des conditions propices à l'apparition d'autres troubles, qu'il est important de pouvoir empêcher.

L'un des buts de l'invention est de remédier à cet inconvénient, en proposant un dispositif permettant d'adapter automatiquement au moment de l'implantation le dispositif, avec ou sans commutation automatique de mode, de la manière la plus appropriée à la pathologie cardiaque du patient, notamment selon que sa conduction auriculo-ventriculaire naturelle est préservée ou non.

L'invention propose à cet effet, essentiellement, de coupler la fonctionnalité AAISafeR à une fonctionnalité de détection automatique de l'implantation. Cette fonctionnalité est par exemple exposée, en tant que telle, dans le EP-A-1 438 985 (ELA Medical), qui décrit un dispositif pourvu de moyens permettant d'analyser les caractéristiques de forme des impulsions recueillies sur les diverses bornes du boîtier et d'en déduire si le dispositif est, ou non, équipé d'une sonde, et s'il a, ou non, été introduit dans le site d'implantation, c'est-à-dire dans l'incision ou "poche" dans laquelle le chirurgien prévoit de placer le générateur.

Plus précisément, l'objet de l'invention est un dispositif d'un type général, en lui-même connu d'après le EP-A-1 550 480 précité, comprenant les éléments indiqués dans le préambule de la revendication 1.

De façon caractéristique de l'invention, le dispositif comporte en outre les éléments énoncés dans la partie caractérisante de la revendication 1.

Les sous-revendications visent des formes particulières de mise en oeuvre préférentielles.

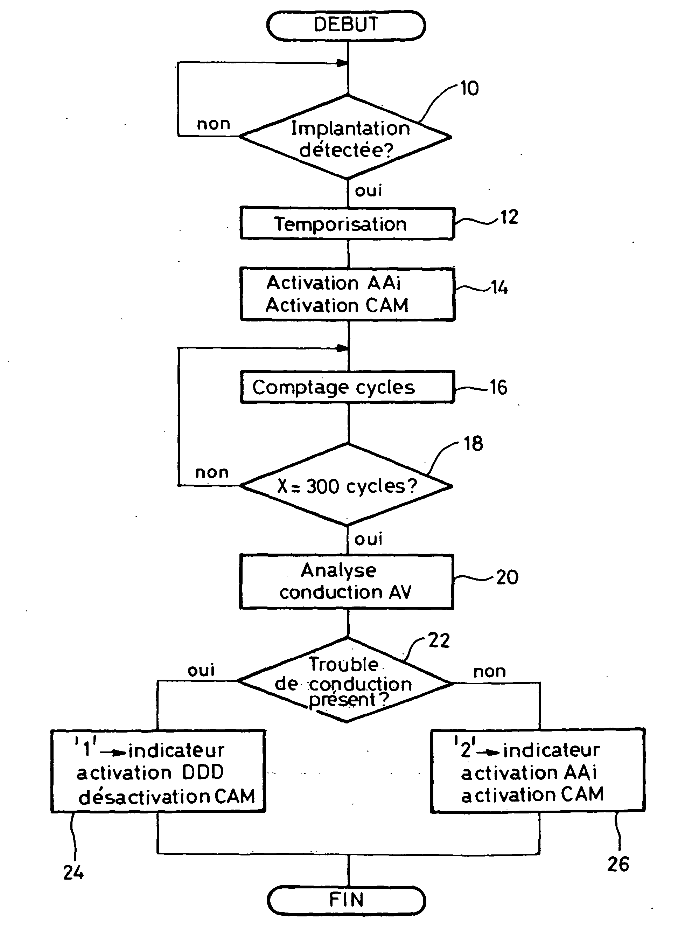

On va maintenant décrire un exemple de mise en oeuvre du dispositif de l'invention, en référence à la figure unique qui est un organigramme des différentes étapes mises en oeuvre par le dispositif de l'invention.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony* et *ELA Rhapsody.* Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Avant toute chose (étape 10), le dispositif détecte si l'implantation a été opérée ou non. Cette détection est opérée par diverses techniques en elles-mêmes connues, par exemple par l'analyse de caractéristiques de forme des impulsions recueillies sur les bornes du générateur, comme cela est enseigné par le EP-A-1 438 985 précité. Cette détection peut être également opérée par d'autres moyens, notamment par scrutation des variations de consommation de la pile du générateur. Ces techniques de détection permettent de détecter non seulement la connexion d'une sonde au générateur du stimulateur et le type de sonde (monopolaire ou bipolaire), mais également de détecter l'implantation effective, c'est-à-dire l'introduction du générateur, équipé de la sonde, dans le site d'implantation (incision ou "poche") où le chirurgien prévoit de placer le boîtier du générateur, configuration où le boîtier est en contact avec les tissus du patient et peut servir de référence de potentiel.

Lorsque ces conditions sont remplies (raccordement de la sonde et implantation effective), le dispositif peut être activé pour le rendre pleinement fonctionnel.

Avantageusement, cette activation n'est pas opérée immédiatement, mais après écoulement d'une temporisation (étape 12) pour éviter les perturbations de l'environnement opératoire sur le rythme cardiaque du patient et les signaux recueillis, et tenir compte d'un éventuel réajustement par le praticien du générateur dans la poche d'implantation qui aurait pour effet d'interrompre provisoirement le contact avec les tissus du patient.

Cette temporisation, qui peut durer par exemple jusqu'à deux heures après détection de l'implantation, n'est cependant pas indispensable. Elle peut être programmée en usine, ou par le praticien implanteur.

L'étape suivante consiste à rendre le dispositif fonctionnel, en activant la fonctionnalité "AAISafeR", c'est-à-dire en plaçant le dispositif en mode AAI et en activant la commutation automatique de mode (indépendamment de la configuration initiale du dispositif, DDD ou AAISafeR, opérée en usine).

Au départ, le fonctionnement du stimulateur est un fonctionnement en mode AAI avec surveillance de l'activité ventriculaire : l'algorithme recherche la présence ou l'absence d'une activité ventriculaire, qui dans ce dernier cas pourrait laisser suspecter un BAV, de manière à éventuellement basculer en mode DDD de stimulation double chambre avec association auriculo-ventriculaire, c'est-à-dire avec calcul et application d'un délai auriculo-ventriculaire pour la stimulation contrôlée du ventricule.

Pour fiabiliser cette recherche, l'analyse doit être réalisée dans des conditions sinusales stables, au repos. En conséquence, la période d'analyse (nombre de cycles ou période de temps) exclut les périodes en trouble de rythme auriculaire ou ventriculaire, en tachycardie sinusale au-delà d'une fréquence donnée (typiquement 100 cpm) ou en phase d'exercice, cette phase étant par exemple déterminée par des capteurs physiologiques.

Au terme d'un certain nombre de cycles de fonctionnement en rythme sinusal lent, par exemple 1500 cycles, ou au terme d'une période de temps donnée, par exemple 15 minutes (étapes 16 et 18), le dispositif opère une analyse de la conduction auriculo-ventriculaire (étape 20). Cette analyse peut être opérée par divers moyens en eux-mêmes connus, notamment :
- comptage du nombre de commutation AAI→DDD depuis l'activation de la fonctionnalité AAISafeR, ou
- comptage, pendant cette même durée, du nombre de cycles cardiaques avec stimulation ventriculaire.

Si le nombre de commutations AAI-DDD est supérieur à un seuil S1, ou si le pourcentage de cycles avec stimulation ventriculaire est supérieur à un seuil S2 donné, alors le dispositif conclut à la présence d'un trouble de la conduction ; dans le cas contraire, il conclut à l'absence d'un tel trouble.

La programmation définitive du mode de stimulation intervient à l'issue du résultat (étape 22) de cette analyse de la conduction auriculo-ventriculaire :
- en présence d'un trouble de la conduction (étape 24), le mode de stimulation retenu pour le patient est le mode DDD permanent, la commutation automatique de mode étant désactivée. Un indicateur est positionné à '1',
- en l'absence de trouble de conduction (étape 26), la fonctionnalité AAISafeR est pleinement activée, c'est-à-dire que la dispositif est mis en mode AAI avec activation de la commutation automatique de mode ; l'indicateur est positionné à '2'.

L'indicateur permettra de délivrer un message au médecin traitant lorsque celui-ci verra le patient pour la première fois après l'implantation, ce message étant par exemple :
- *« l'appareil fonctionne en DDD en raison des troubles de conduction AV que votre patient a présenté au moment de l'implantation »* (si l'indicateur est à '1'), ou
- « l'appareil fonctionne en AAISafeR car votre patient a présenté une conduction AV préservée au moment de l'implantation » (si l'indicateur est à '2').

## Revendications

1. Un dispositif médical implantable actif, notamment un stimulateur cardiaque, défibrillateur et/ou cardioverteur, comprenant :
- des moyens de détection d'événements auriculaires et ventriculaires spontanés,
- des moyens de stimulation ventriculaire et auriculaire,
- des moyens aptes à faire fonctionner le dispositif en mode AAI avec détection ventriculaire,
- des moyens pour faire fonctionner le dispositif en mode DDD, et
- des moyens de commutation de mode, pour commander en fonction de critères prédéterminés le basculement du mode AAI en mode DDD, et inversement le retour du mode DDD au mode AAI,
dispositif **caractérisé en ce qu'**il comprend :
- des moyens de détection automatique d'implantation, et
- des moyens d'adaptation automatique de mode, activés sur détection de l'implantation (10) par les moyens de détection automatique d'implantation, ces moyens d'adaptation automatique de mode comprenant des moyens pour, successivement :
• placer (14) le dispositif en mode AAI avec détection ventriculaire et activer les moyens de commutation de mode, indépendamment de la configuration initiale du dispositif,
• analyser (20) la conduction atrio-ventriculaire de manière à détecter un trouble éventuel de la conduction pendant une période de rythme sinusal lent,
• programmer définitivement le mode de stimulation à l'issue du résultat de l'analyse de la conduction atrio-ventriculaire, en :
• en cas de trouble de conduction détecté (24), positionnant le dispositif en mode DDD et inhibant les moyens de commutation de mode,
• en l'absence de trouble de conduction détecté (26), positionnant le dispositif en mode AAI avec détection ventriculaire et activant les moyens de commutation de mode,
de manière à adapter automatiquement le dispositif au moment de l'implantation, avec ou sans commutation automatique de mode, de la manière, la plus appropriée à la pathologie cardiaque du patient, notamment selon que sa conduction auriculo-ventriculaire naturelle est préservée ou non.

2. Le dispositif de la revendication 1, où les moyens d'adaptation automatique de mode comprennent des moyens pour positionner un indicateur, conservé dans une mémoire permanente du dispositif, sélectivement en fonction de la présence ou de l'absence de trouble de conduction détecté.

3. Le dispositif de la revendication 1, comprenant des moyens pour appliquer un délai (12) à l'activation des moyens d'adaptation automatique de mode après détection de l'implantation (10) par les moyens de détection automatique d'implantation.

4. Le dispositif de la revendication 1, où les moyens pour analyser la conduction atrio-ventriculaire (20) comprennent des moyens pour déterminer la présence d'un trouble de conduction lorsque, sur une durée prédéterminée ou un nombre de cycles cardiaques prédéterminé, le nombre de commutations de mode de AAI vers DDD excède un premier seuil prédéterminé.

5. Le dispositif de la revendication 1, où les moyens pour analyser la conduction atrio-ventriculaire (20) comprennent des moyens pour déterminer la présence d'un trouble de conduction lorsque, sur une durée prédéterminée ou un nombre de cycles cardiaques prédéterminé, le pourcentage de cycles ventriculaires stimulés excède un deuxième seuil prédéterminé.

6. Le dispositif de l'une des revendications 4 ou 5, où les moyens pour analyser la conduction atrio-ventriculaire (20) opèrent l'analyse de la conduction en excluant de ladite durée prédéterminée ou dudit nombre de cycles cardiaques prédéterminé, les périodes de présence de troubles du rythme auriculaire et ventriculaire, les phases de tachycardie sinusale au-delà d'une valeur prédéterminée de fréquence cardiaque, et les phases d'exercice identifiés par des capteurs physiologiques.

## Claims

1. An active implantable medical device, in particular a cardiac pacemaker, defibrillator and/or cardioverter, comprising
- means for detecting spontaneous atrial and ventricular events,
- means for ventricular and atrial stimulation,
- means adapted for operating said device in the AAI mode with ventricular detection,
- means for operating said device in the DDD mode, and
- mode switching means for controlling as a function of predetermined criteria the changeover from the AAI mode to the DDD mode, and conversely the reversal from the DDD mode to the AAI mode,
said device being **characterized in that** it comprises:
- means for automatic implantation detection,
- means for automatic mode adaptation, that are activated following detection of the implantation (10) through the means for automatic implantation detection, wherein said automatic adaptation means comprise means for successively
• putting in place (14) the device in the AAI mode with ventricular detection and activating the mode switching means independently from the initial configuration of the device,
• analyzing (20) the atrio-ventricular conduction so as to detect a possible conduction disturbance during a slow sinusal rhythm period,
• definitively programming the stimulation mode at receiving results of the atrio-ventricular conduction analysis, through
• in case of a detected conduction disturbance (24), positioning the device in the DDD mode and inhibiting the mode switching means,
• in the absence of detected conduction disturbance (24), positioning the device in the AAI mode with ventricular detection and activating the mode switching means,
in order to automatically adapt the device at the implantation moment, with or without automatic mode switching, in the manner most suitable for patient's cardiac pathology, especially depending on whether his natural atrio-ventricular conduction is maintained or not.

2. The device according to claim 1, wherein the automatic mode adaptation means comprise means for positioning an indicator stored in a permanent memory of the device, selectively as a function of the presence or the absence of a detected conduction disturbance.

3. The device according to claim 1, comprising means for delaying (12) the activation of the mode automatic adaptation means after detecting the implantation (10) thanks to the implantation automatic detection means.

4. The device according to claim 1, wherein the atrio-ventricular conduction analysis means (20) comprise means for determining the presence of a conduction disturbance when during a predetermined duration or during a predetermined number of cardiac cycles the number of mode switchings from AAI to DDD exceeds a first predetermined threshold.

5. The device according to claim 1, wherein the atrio-ventricular conduction analysis means (20) comprise means for determining the presence of a conduction disturbance when during a predetermined duration or during a predetermined number of cardiac cycles the percentage of stimulated ventricular cycles exceeds a second predetermined threshold.

6. The device according to one of claims 4 or 5, wherein the atrio-venticular conduction analyzing means (20) perform the conduction analysis excluding said predetermined duration or said predetermined number of cardiac cycles, the periods of presence of atrial or ventricular rhythm disturbances, the sinusal tachycardia phases above a predetermined value of the heart rate, and the exercise phases identified by physiological sensors.

## Patentansprüche

1. Eine aktive implantierbare medizinische Vorrichtung, insbesondere ein Herzschrittmacher, ein Defibrillator und/oder ein Kardioverter, umfassend :
- Mittel zur Detektion von spontanen Vorhof- und Kammerereignissen,
- Mittel zur Herzkammer- und Herzvorhofstimulation,
- Mittel, die geeignet sind, die Vorrichtung in AAI-Modus mit Herzkammerdetektion zu betreiben,
- Mittel zum Betreiben der Vorrichtung in DDD-Modus, und
- Mittel zur Modusumschaltung, um in Abhängigkeit von vorgegebenen Kriterien den Wechsel vom AAI-Modus zum DDD-Modus und umgekehrt den Wechsel vom DDD-Modus zum AAI-Modus zu befehlen,
**dadurch gekennzeichnet, dass** sie umfasst :
- Mittel zur automatischen Detektion einer Implantation, und
- nach der Detektion der Implantation (10) durch die Mittel zur automatischen Detektion einer Implantation aktivierte Modusanpassungsmittel, wobei genannte Modusanpassungsmittel Mittel umfassen, um nacheinander
• die Vorrichtung in AAI-Modus mit Kammerdetektion zu legen (14) und die Mittel zur Modusumschaltung unabhängig von der Anfangskonfiguration der Vorrichtung zu aktivieren,
• die atrioventrikulare Leitung zu analysieren (20, um eine mögliche Leitungsstörung während einer langsamen Sinusrhythmusperiode zu erfassen,
• endgültig den Stimulationssmodus nach dem Empfang des Ergebnisses der Analyse der atrioventrikularen Leitung zu programmieren
• im Falle einer detektierten Leitungsstörung (24) durch Positionierung der Vorrichtung in DDD-Modus und Inhibierung der Modusumschaltungsmittel,
• in Abwesenheit von einer detektierten Leitungsleitung (24) durch Positionierung der Vorrichtung in AAI-Modus mit Herzkammerdetektion und Aktivation der Modusumschaltungsmittel,
um automatisch die Vorrichtung zur Zeit der Implantation ohne oder mit automatischer Modusumschaltung in der für die Herzpathologie des Patienten geeignetsten Weise anzupassen, insbesondere je nachdem, ob seine natürliche atrioventrikulare Leitung erhalten bleibt oder nicht.

2. Die Vorrichtung gemäss Anspruch 1, wobei die Mittel zur automatischen Modusanpassung Mittel umfassen, um einen in einem permanenten Speicher aufbewahrten Indikator selektiv in Abhängigkeit von der An- oder Abwesenheit einer detektierten Leitungsstörung zu positionieren.

3. Die Vorrichtung gemäss Anspruch 1, umfassend Mittel zur Verzögerung (12) der Aktivation der Mittel zur automatischen Modusanpassung nach der Detektion der Implantation (1) durch die Mittel zur automatischen Detektion einer Implantation.

4. Die Vorrichtung gemäss Anspruch 1, wobei die Mittel zur Analyse der atrioventrikularen Leitung (20) Mittel umfassen, um die Anwesenheit einer Leitungsstörung zu bestimmen, als die Zahl der Umschaltungen vom AAI-Modus zum DDD-Modus während einer vorgegebenen Zeitdauer oder einer vorgegebenen Zahl von Herzzyklen eine erste vorgegebene Schwelle überschreitet.

5. Die Vorrichtung gemäss Anspruch 1, wobei die Mittel zur Analyse der atrioventrikularen Leitung (20) Mittel umfassen, um die Anwesenheit einer Leitungsstörung zu bestimmen, als der Prozentsatz der stimulierten Kammerzyklen während einer vorgegebenen Zeitdauer oder einer vorgegebenen Zahl von Herzzyklen eine zweite vorgegebene Schwelle überschreitet.

6. Die Vorrichtung gemäss einem der Ansprüche 4 oder 5, wobei die Mittel zur Analyse der atrioventrikularen Leitung (20) die Analyse der Leitung ausschliessend genannter vorgegebenen Zeitdauer oder genannter Zahl von Herzzyklen, der Perioden des Vorhof- und Kammerrhythmusstörungen, der Sinustachykardiephasen über einer vorgegebenen Herzfrequenzwert, und der durch physiologischen Sensoren erkannten Betätigungsphasen ausführen.
